# EUROPÄISCHE PATENTSCHRIFT

(11) **EP 2 222 654 B1**
(45) Veröffentlichungstag und Bekanntmachung des Hinweises auf die Patenterteilung: **20.03.2013**
(21) Anmeldenummer: 08865833.1
(22) Anmeldetag: 01.12.2008
(51) Int. Cl.: C07D 263/18, C07D 263/52, C07D 317/34, C07D 317/72, A23L 1/27, A23L 1/305

(54) **2-METHYLTHIOETHYL-SUBSTITUIERTE HETEROCYCLEN ALS FUTTERMITTELADDITIVE**
2-METHYLTHIOETHYL-SUBSTITUTED HEYRROCYCLES AS FEED ADDITIVES
HÉTÉROCYCLÈNE SUBSTITUÉ PAR UN GROUPE 2-MÉTHYLTHIOÉTHYLE EN TANT QU'ADDITIF POUR L'ALIMENTATION ANIMALE

(30) Priorität: 21.12.2007 DE 102007062199
(43) Veröffentlichungstag der Anmeldung: 01.09.2010
(73) Patentinhaber: Evonik Degussa GmbH, 45128 Essen (DE)
(72) Erfinder: KOBLER, Christoph, 63755 Alzenau (DE); ROTH, Philipp, 63456 Hanau (DE); WECKBECKER, Christoph, 63584 Gründau-Lieblos (DE); HUTHMACHER, Klaus, 63571 Gelnhausen (DE)
(86) Internationale Anmeldenummer: PCT/EP2008/066525
(87) Internationale Veröffentlichungsnummer: WO 2009/080446

(56) Entgegenhaltungen:
- WO-A-00/28835
- HIDETOSHI TOKUYAMA ET AL: "Reduction of ethanethiol esters to aldehydes" SYNTHESIS, Bd. 8, 2002, Seiten 1121-1123, XP002517494

## Beschreibung

### Einleitung

Die vorliegende Erfindung betrifft neue 2-methylthioethylsubstituierte Heterocyclen und deren Derivate sowie deren Herstellung und deren Verwendung als Futtermitteladditive, insbesondere für die Ernährung von Nutztieren, wie z.B. Hühnern, Schweinen, Wiederkäuern, aber auch von Fischen und Krustentieren (Meerestieren).

### Stand der Technik

Essentielle Aminosäuren wie Methionin, Lysin oder Threonin sind als Futtermitteladditive sehr wichtige Bestandteile der Tierernährung. Deren Supplementierung ermöglicht zum einen ein schnelleres Wachstum der Tiere, zum anderen aber auch effizientere Verwertung des Futters. Dies stellt einen großen wirtschaftlichen Vorteil dar. Die Märkte für Futtermitteladditive sind von großer industrieller und wirtschaftlicher Bedeutung. Zudem sind sie starke Wachstumsmärkte, was nicht zuletzt auf die steigende Bedeutung von Ländern wie beispielsweise China und Indien zurückzuführen ist.

Aus WO 2004008874 ist unter anderem bekannt, dass Methionin (2-Amino-4-methylthiobuttersäure) für vielen Tierarten die erste limitierende Aminosäure darstellt. So ist beispielsweise bei Milchkühen die effiziente Milchproduktion hinsichtlich der Menge und Qualität sehr stark von einer ausreichenden Zufuhr von Methionin abhängig. Der Methioninbedarf von Hochleistungsmilchkühen kann dabei nicht durch das im Pansen gebildete Mikrobeneiweiß bzw. durch im Pansen nicht abgebautes Eiweiß aus dem Futter gedeckt werden (Graulet et al., J. Animal and Feed Sciences (2004), 269). Es ist daher vorteilhaft, Methionin dem Futter zu supplementieren, um die Wirtschaftlichkeit der Milchproduktion und die Qualität der Milch zu erhöhen.

Bei monogastrischen Tieren wie z.B. Geflügel und Schweinen wird üblicherweise D,L-Methionin und das Methionin-Hydroxy-Analog (MHA), mit der chemischen Bezeichnung D,L-2-Hydroxy-4-methylthiobuttersäure (HMB), als Futtermitteladditiv verwendet. Dadurch wird die verfügbare Menge an L-Methionin im Organismus erhöht, die dann dem Tier zum Wachstum zur Verfügung stehen kann.

Im Gegensatz dazu ist die Supplementierung des Futters mit Methionin bei Wiederkäuern nicht effektiv, da die Hauptmenge im Pansen (Rumen) der Wiederkäuer durch Mikroben abgebaut wird. Aufgrund dieses Abbaus gelangt daher nur ein Bruchteil des zugeführten Methionins in den Dünndarm des Tiers, wo im Allgemeinen die Absorption des Methionins ins Blut erfolgt.

In WO 99/04647 wird die Verwendung von MHA für Wiederkäuer beschrieben. Darin wird behauptet, dass MHA nur zum Teil im Pansen abgebaut wird und daher mindestens 20-40% des supplementierten MHAs nach Absorption im Dünndarm in den Stoffwechsel gelangen können. In zahlreichen anderen Publikationen wird dagegen die Wirkungsweise von MHA beim Wiederkäuer unterschiedlich diskutiert. So wird beispielsweise in WO 200028835 beschrieben, dass MHA nur dann den Pansen erfolgreich passieren und schlussendlich zur Absorption in den Dünndarm gelangen kann, wenn MHA in sehr großen Mengen von 60-120g/Tag/Tier verabreicht wird. Dadurch ist jedoch eine Wirtschaftlichkeit nicht mehr gegeben.

Damit dem Wiederkäuer Methioninprodukte wie D,L-Methionin bzw. rac-MHA mit hoher Effizienz zur Verfügung stehen, muss eine vor dem Pansenabbau geschützte Form eingesetzt werden. Die Herausforderung ist hierbei, ein geeignetes Methioninprodukt aufzufinden, das dem Methionin eine möglichst hohe Pansenstabilität verleiht und trotzdem eine hohe und effiziente Absorption des Methionins im Darm gewährleistet. Dabei gibt es mehrere Möglichkeiten, dem D,L-Methionin oder rac-MHA diese Eigenschaften zu verleihen:

### a)Physikalischer Schutz:

Durch Anbringung einer geeigneten Schutzschicht bzw. Verteilung des Methionins in einer Schutzmatrix kann eine hohe Pansenstabiliät erreicht werden. Dadurch kann das Methionin den Pansen praktisch ohne Verlust passieren. Im weiteren Verlauf wird die Schutzschicht dann z. B. im Labmagen durch saure Hydrolyse geöffnet oder entfernt und das freiwerdende Methionin kann dann im Dünndarm vom Tier absorbiert werden.

Die Schutzschicht bzw. -matrix kann aus einer Kombination von mehreren Substanzen wie z.B. Lipiden, anorganische Materialien und Kohlenhydraten bestehen. Beispielsweise folgende Produktformen sind kommerziell erhältlich:
i) Met-Plus™ von Nisso America ist ein lipidgeschütztes Methionin mit einem D,L-Methioningehalt von 65%. Die Schutzmatrix besteht aus den Calciumsalzen langkettiger Fettsäuren wie z.B. Laurinsäure. Als Konservierungsstoff dient butyliertes Hydroxytoluol.
ii) Mepron® M85 von Degussa AG ist ein kohlenhydratgeschütztes Methionin, das einen Kern aus D,L-Methionin, Stärke und Stearinsäure besitzt. Als Schutzschicht wird Ethylcellulose verwendet. Das Produkt hat einen Gehalt von 85% D,L-Methionin.
iii) Smartamine™ M von Adisseo ist ein polymergeschütztes Methionin. Die Pellets enthalten neben Stearinsäure mind. 70% D,L-Methionin. Die Schutzschicht enthält Vinylpyridin-Styrol-Copolymer.

Obwohl der physikalische Schutz den.mikrobiellen Abbau des Methionins im Pansen verhindert und dadurch die Zufuhr und Verwertung von Methionin im Tier erhöht werden kann, gibt es einige gravierende Nachteile.

Die Herstellung bzw. die Beschichtung von Methionin stellt meist ein technisch kompliziertes und aufwendiges Verfahren dar und ist daher teuer. Zudem kann die oberflächliche Beschichtung der fertigen Pellets leicht durch mechanische Belastung und Abrieb während der Futterverarbeitung beschädigt werden, was zur Verminderung bzw. bis zum vollständigen Verlust des Schutzes führen kann. Deshalb ist es auch nicht möglich, die geschützten Methioninpellets in ein größeres Mischfutterpellet zu verarbeiten und neu zu pelletieren, da dadurch wiederum die schützende Schicht durch die mechanische Beanspruchung aufbrechen würde. Dies schränkt die Verwendung solcher Produkte stark ein, da die Mischfutterpelletierung eine weit verbreitete Methode der Futterverarbeitung darstellt.

### b) Chemischer Schutz:

Eine erhöhte Pansenstabilität von Methionin kann neben den rein physikalischen Schutzmöglichkeiten auch durch Modifikation der chemischen Struktur, beispielsweise durch Veresterung der Carbonsäuregruppe, erreicht werden. Zurzeit sind folgende Produkte kommerziell erhältlich oder in der Literatur beschrieben:
i) Methioninester wie z.B. D,L-tert-Butylmethionin: Die Ester wurden getestet und zeigten nur einen moderate Pansenstabilität (Loerch und Oke; "Rumen Protected Amino Acids in Ruminant Nutrition" in "Absorption and Utilization of Amino Acids" Vol. 3, 1989, 187-200, CRC Press Boca Raton, Florida). Für D,L-tert-Butylmethionin wurde dagegen in WO 0028835 eine Biowertigkeit von 80% veröffentlicht.
ii) Metasmart™ von Adisseo ist der racemische iso-Propylester von MHA (HMBi). Diese Verbindung wird auch unter dem Trademark "Sequent" von der amerikanischen Firma

Novus vermarktet. In WO 00/28835 wurde eine Biowertigkeit von mindestens 50% für HMBi bei Wiederkäuern veröffentlicht. Dabei spielt vor allem die überraschend schnelle Absorption des hydrophoben HMBi's über die Pansenwand eine entscheidende Rolle. Der Ester kann dann im Blut zu MHA hydrolysiert und nach Oxidation und anschließender Transaminierung zum L-Methionin umgewandelt werden. Im Patent EP 1358805 wurde eine vergleichbare Biowertigkeit für HMBi publiziert. Bei diesen Untersuchungen war HMBi auf einem porösen Träger aufgebracht. In einer weiteren Veröffentlichung wurde von der Europäischen Kommission berichtet, dass wiederum ca. 50% HMBi über die Pansenwand absorbiert werden (European Commission: Report of the Scientific Committee on Animal Nutrition on the Use of HMBi; 25 April 2003). Graulet et al. veröffentlichte 2004 im Journal of Animal and Feed Science (269), dass durch die lipophilen Eigenschaften der iso-Propylgruppe von HMBi eine bessere Diffusion über die Pansenwand ermöglicht wird.

Zur Herstellung von HMBi sind zwei verschiedene Verfahren veröffentlicht worden. So kann HMBi entweder direkt in einer Stufe aus dem entsprechenden Cyanhydrin (WO 00-59877) dargestellt werden. Die Veresterung zum iso-Propylester erfolgt dabei *in situ,* ohne zuvor MHA isolieren zu müssen. Ein anderes Verfahren verestert dagegen reines MHA mit *iso*Propanol (WO 01-58864 und WO 01-56980). In beiden Fällen wird zur Synthese Blausäure verwendet, die teuer ist und zudem ein großes Gefahrenpotenzial in sich birgt.

Hidetoshi Tokuyama et al.: ("Reduction of ethanethiol esters to aldehydes" Synthesis, Bd. 8, 2002, Seiten 1121-1123), offenbart in Tabelle 1 lediglich einen heterozyklischen Ethanthiolester, jedoch keine Verwendung der offenbarten Verbindung in Zusammenhang mit Futtermittelzusätzen.

Auch der Bereich des Aquafarming (Food and Agriculture Organization of the United Nation (FAO) Fisheries Department "State of World Aquaculture 2006", 2006, Rome. International Food Policy Research Institute (IFPRI) "Fish 2020: Supply and Demand in Changing Markets", 2003, Washington, D.C.) hat in den letzten Jahren vermehrt an Bedeutung gewonnen. Die Aufzucht von genießbaren Salz- und Süßwassertieren, insbesondere von Fischen und Krustentieren erfor-dert ebenfalls besondere Produktformen für die Versorgung mit Methionin.

Die Versorgung von Fischen und Krustentieren, die kommerziell in Aquakulturen gehalten werden, erfordert eine entsprechend geschützte Produktform, zum einen, damit das Produkt während der Fütterung in der wässrigen Umgebung hinreichend stabil bleibt und zum anderen, damit das schließlich vom Tier aufgenommene Methionin-Produkt im tierischen Organismus optimal verwertet werden kann.

### Aufgabe der Erfindung

Eine generelle Aufgabe war es, ein Futtermittel bzw. einen Futtermittelzusatzstoff in der Tierernährung auf Basis neuer Methioninersatzstoffe bereitzustellen.

Vor dem Hintergrund der Nachteile des Standes der Technik war es vor allem die Aufgabe, ein chemisch geschütztes Methioninprodukt für Nutztiere bereitzustellen. Insbesondere sollte dieses Produkt pansenstabil sein zur Verwendung für Wiederkäuer, im speziellen für Milchkühe. Auch sollte das Produkt möglichst geeignet sein zur Verwendung bei der Ernährung von Fischen und Krustentieren in Aquakulturen. Auf diese Weise sollte den Tieren neben D,L-Methionin und MHA eine weitere effiziente Methioninquelle zur Verfügung gestellt werden, welche möglichst die Nachteile der bekannten Produkte nicht oder nur in verringertem Umfang aufweist.

Eine weitere Aufgabe war es, ein Futtermittel bzw. einen Futtermittelzusatzstoff mit sehr hoher Biowertigkeit aufzufinden, das gute Handhabbarkeit und Lagerfähigkeit sowie Stabilität unter den üblichen Bedingungen der Mischfutterverarbeitung, insbesondere der Pelletierung aufweisen sollte. Im Falle der Wiederkäuer hätte ein solches Produkt den Vorteil einer deutlich einfacheren und standardisierten Mischfutterverarbeitung/-bereitstellung, so dass damit die Wirtschaftlichkeit aber auch die Qualität der Milchproduktion erhöht würde.

### Beschreibung der Erfindung

Gelöst werden diese sowie weitere nicht explizit genannte Aufgaben, die jedoch aus den hierin diskutierten Zusammenhängen ohne weiteres ableitbar oder erschließbar sind durch die erfindungsgemäßen heterocyclischen Verbindungen und deren Derivate gemäß Formel I bzw. Formel II insbesondere deren Verwendung als Futtermittel, vorzugsweise für Hühner, Schweine, Wiederkäuer, Fische und Krustentiere.

Gegenstand der vorliegenden Erfindung ist daher eine chemische Verbindung der allgemeinen Formel (**I**) oder (**II**), 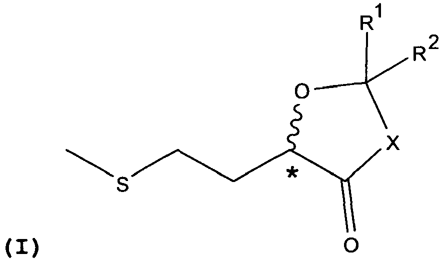 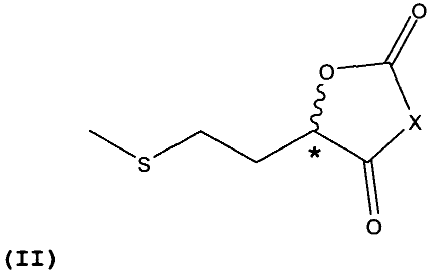 wobei X = O oder NR ist und R = H, ein ggf. verzweigtes C₁-C₆-Alkyl, C₃-C₆-Cycloalkyl, Aryl, insbesondere Phenyl, oder Aralkyl, insbesondere Benzyl, ist und wobei R¹, R² gleich oder verschieden ist und jeweils H, ein ggf. verzweigtes C₁-C₆-Alkyl, C₃-C₆-Cycloalkyl, Allyl-, Aryl, insbesondere Phenyl, oder Aralkyl, insbesondere Benzyl, oder R¹ und R² zusammen eine ggf. C₁-C₆-alkylsubstituierte C₂- bis C₆-Alkylengruppe ist.

Die Vorteile der Verbindung **I** liegt z.B. darin, dass sie für R¹, R² = H bzw. = Niedrigalkylrest wie Methyl, Ethyl, n-Propyl flüssige, wasserklare, farblose Komponenten darstellen. Zum anderen sind die Komponenten der Formel **I** frei von dimeren und oligomeren Nebenprodukten ganz im Gegensatz zur kommerziell erhältlichen 2-Hydroxy-4-methylthioethylbuttersäure (MHA-Monomer). Diese steht im Gleichgewicht mit ihren dimeren und höheren oligomeren Estern (Kondensationsprodukte), welche eine deutlich niedrigere Bioverfügbarkeit als MHA-Monomer oder D,L-Methionin selbst aufweisen. MHA wird daher ähnlich wie die analoge Milchsäure als 88proz. wässrige Lösung in den Handel gebracht, um das Gleichgewicht in Richtung des gewünschten Monomers zu beeinflussen.

Die erfindungsgemäßen Komponenten müssen hingegen nicht mit Wasser verdünnt werden, so dass der reine Wirkstoff zur Verfügung steht. Darüberhinaus sind sie leicht destillierbar, besonders im Fall von R¹, R² = H, Methyl, Ethyl ,n-Propyl, so dass auf eine technisch einfach durchführbare Weise eine fast 100%ige Reinheit dieser neuen Stoffe erreicht werden kann, was einen außerordentlichen verfahrenstechnischen und damit auch wirtschaftlichen Vorteil darstellt.

Die flüssigen Verbindungen **I** bzw. **II**, jeweils mit X = O, können direkt als Flüssigfuttermittelzusatzstoff verwendet werden, was für bestimmte Anwendungen Vorteile bietet, insbesondere dann, wenn in Mischfutterbetrieben Flüssigdosiersysteme für sogenannte Mikrokomponenten bereits vorhanden sind. Wahlweise können diese Komponenten jedoch auch auf feste Träger aufgebracht werden, die anorganischer oder organischer Natur sein können und futtermitteltauglich sein sollten und so auf einfache Weise ein fester Futtermittelzusatzstoff erzeugt werden, der dort, wo nur Feststoffdosiersysteme zur Verfügung stehen, genauso leicht gehandhabt werden kann, wie z.B. D,L-Methionin als klassisches festes Futtermitteladditiv.

Solche anorganischen Träger können sein Kieselsäuren, wie z.B. Sipernat von Evonik-Degussa, oder Silikate, sowie Aluminiumoxide oder Zeolithe, z.B. Calcium-, Natrium- oder Natriumaluminiumsilikat, oder Metallcarbonate wie Magnesium-, Calcium- oder Natriumcarbonat, einzeln oder in Mischung zweier oder mehrerer derartiger Trägerstoffe.

Solche organischen Träger können beispielsweise sein Alginate, Stearate, Stärken und Gummis. Bevorzugt sind Calcium-, Natrium- oder Aluminiumalginat, Calcium- oder Natriumstearat, Maisstärke oder Gummi arabicum einzeln oder in Mischung zweier oder mehrerer derartiger Trägerstoffe.

Auf diese Weise kann auch gezielt eine niedrigere Konzentration als 100 % der erfindungsgemäßen Komponente eingestellt werden, sofern dies erwünscht ist.

Bevorzugt werden Verbindungen der Formel **I** bei denen X = O ist, da diese sowohl Acetale als auch Ester darstellen und hier bei der Hydrolyse im Organismus direkt monomeres MHA entsteht, welches anschließend verstoffwechselt werden kann. Dabei wird gleichzeitig die entsprechende Carbonylverbindung R¹R²C=O freigesetzt.

Bevorzugt sind hier Verbindungen der Formel **I**, bei denen R¹ und R² jeweils ein ggf. verzweigtes C₁-C₆-Alkyl ist. Aus physiologischen Gründen ist hier die Verbindung **4** mit R¹=R²= CH₃ besonders bevorzugt, da bei der MHA-Freisetzung lediglich Aceton entsteht, das physiologisch unbedenklich ist. Aufgrund der geringen Konzentration von typischerweise 0,1 bis 0,5 Gew.% Methioninäquivalent im Mischfutter sind aber auch andere Reste R¹,R² bzw. die bei Hydrolyse zum MHA entsprechend freigesetzten Carbonylverbindungen vertretbar.

Weiter bevorzugt sind deshalb die Verbindung **2** (vgl. Beispiele), mit R¹ = R² = H und Verbindung **6** mit R¹ = H und R² = tert-Butyl. Auch Verbindung I mit X = O und R¹ = H, R² = Phenyl ist hier bevorzugt, da bei deren Hydrolyse Benzaldehyd entsteht, der auch in pflanzlichen Produkten wie Bittermandeln vorkommt. Bei der Hydrolyse von **2** entsteht als Carbonylverbindung Formaldehyd, der leicht zu Formiat weiteroxidiert wird, welches selbst als Futtereinsatzstoff Bedeutung hat.

Ebenfalls noch bevorzugt ist eine Verbindung **7** der allgemeinen Formel **I**_{,} bei der R¹ und R² zusammen = (CH₂)₅ ist, so dass bei deren Hydrolyse Cyclohexanon freigesetzt wird.

Bevorzugte Verbindungen im Sinne der vorliegenden Erfindung sind jedoch auch Verbindungen der Formel **I** bei denen X = NH ist. Bei deren Hydrolyse wird neben der entsprechenden Carbonylverbindung R¹R²C=O gleichzeitig Ammoniak freigesetzt. Dieser Ammoniak stellt genau das molare NH₃-Äquivalent dar, das im Organismus für den Metabolismus der erfindungsgemäßen Verbindung **II** zur Aminosäure Methionin gebraucht wird.

Hierbei bevorzugt ist Verbindung **I** bei der X = NH und R¹ = R² = H ist. Bei deren Hydrolyse entsteht als Carbonylverbindung Formaldehyd, der leicht zu Formiat weiteroxidiert wird, welches selbst als Futtereinsatzstoff Bedeutung hat.

Auch bevorzugt ist Verbindung **12** bei der R¹ = H und R² = Phenyl ist. Bei deren Hydrolyse entsteht als Carbonylverbindung Benzaldehyd, der natürlicher Bestandteil von Bittermandeln ist.

Auch bevorzugt sind Verbindungen der Formel **I** mit X = NH, die dadurch gekennzeichnet sind, dass R¹ und R² jeweils ein ggf. verzweigtes C₁-C₆-Alkyl ist.

Hierbei ganz besonders bevorzugt ist Verbindung **10**, bei der R¹ = R² = CH₃ ist und bei deren Hydrolyse lediglich NH₃ und Aceton freigesetzt werden.

Aber auch die Verbindung **13** mit R¹ = CH₃ und R² = C₂H₅ und die Verbindung **14** mit R¹ und R² zusammen = (CH₂)₅ sind neue, interessante Futtermitteleinsatzstoffe.

Darüberhinaus wurde die Verbindung **8** gefunden mit der Formel **II**, bei der X = O ist. Diese Substanz ist bei Raumtemperatur flüssig. Eine Hydrolyse führt direkt zum Monomeren MHA und liefert als Nebenprodukt lediglich CO₂, das sowieso im natürlichen Metabolismus der Lebewesen vorkommt und daher völlig unbedenklich ist. Dies ist von außerordentlichem Vorteil für die Tierernährung.

Das in gleicher Weise interessante Gegenstück dazu ist die Verbindung **15** mit der Formel **II** und X = NH, die einen farblosen Feststoff darstellt. Eine Hydrolyse führt ebenso direkt zum MHA-Monomer (2-Hydroxy-4-methylthioethyl-buttersäure) und liefert neben CO₂ als weiteres Nebenprodukt noch NH₃, was ebenso im natürlichen Metabolismus der Lebewesen vorkommt bzw. wiederum als NH₃-Äquivalent für die Aminosäurebildung aus der Hydroxysäure MHA-Monomer bereitsteht und daher sogar noch Vorteile bieten kann.

Alle erfindungsgemäßen Verbindungen der allgemeinen Formel **I** und **II** sind prinzipiell geeignet für die Verwendung zur Ernährung von Nutztieren, da alle den Grundkörper des Methioninhydroxyanalogen beinhalten, der bei der physiologischen Metabolisierung der Verbindungen als 2-Hydroxy-4-methylthiobutyrat freigesetzt wird und schließlich zu Methionin umgesetzt wird. Weitere Vorteile derartiger chemisch geschützter Methionin-Analoga wurden eingangs bzw. vorstehend beschrieben. Derartige chemisch geschützte Produktformen sind, zum einen während der Fütterung und auch in wässriger Umgebung hinreichend stabil und zum anderen im tierischen Organismus verwertbar. Je nach Tierart und Futtermittelmatrix bzw. Fütterungsbedingungen wird der Fachmann die eine oder andere Komponente bevorzugt in Betracht ziehen.

Derartige Verbindungen können insbesondere Verwendung finden zur Ernährung von Geflügel, von Schweinen, von Wiederkäuern, aber auch zur Ernährung von Fischen oder Krustentieren. Futtermischungen zur Ernährung von Nutztieren enthaltend mindestens eine der Verbindungen der allgemeinen Formel **I** oder **II** sind auch Gegenstand der vorliegenden Erfindung, sowie die entsprechende Verwendung dieser Verbindungen zur Herstellung von Futtermischungen zur Ernährung von Nutztieren.

Auch ein entsprechendes Verfahren zur Herstellung der Verbindungen der allgemeinen Formel **I** oder **II** ist Gegenstand der vorliegenden Erfindung.

Ein solches Verfahren geht aus von einer Verbindung der allgemeinen Formel **III**, 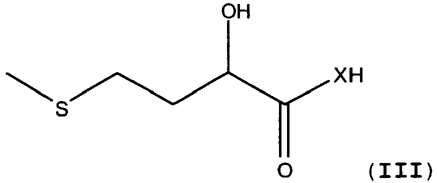 wobei X, R¹ und R² jeweils die oben angegebene Bedeutung haben. Im Falle von X = O steht **III** für die 2-Hydroxy-4-methylthiobuttersäure (Verbindung **3**, MHA-Monomer), welche auch aus einem ihrer Salze , bevorzugt dem Calciumsalz (Verbindung **1**, vgl. Beispiel 1) mit Säure in situ erzeugt werden kann. Im Falle von X= NH steht **III** für 2-Hydroxy-4-methylthiobuttersäure-amid (Verbindung **9**, MHA-Amid), das aus 2-Hydroxy-4-methylthiobuttersäure-nitril nach bekannten Hydrolyseverfahren z.B. mit 55-70-prozentiger Schwefelsäure gewonnen werden kann.

Gegenstand der Erfindung ist somit ein Verfahren zur Herstellung von Verbindungen der Formel **I**, dadurch gekennzeichnet, dass man eine Verbindung der allgemeinen Formel **III** mit einer Carbonylverbindung R¹R²C=O in freier oder in acetalisierter Form ggf. in Anwesenheit eines Lösungsmittels umsetzt. Geeignete Lösungsmittel hierbei sind beispielsweise Toluol oder Chloroform, welche gleichzeitig als Schleppmittel dienen können, sowie Tetrahydrofuran, Dioxan, Methylenchlorid und Dimethylformamid. Besonders vorteilhaft ist es jedoch, die verwendete Carbonylverbindung gleichzeitig als Lösungsmittel einzusetzen, insbesondere dann, wenn es sich um ein Keton handelt, wie beispielsweise im Falle von Aceton oder Methylethylketon. Die überschüssige Carbonylverbindung kann nach Beendigung der Reaktion leicht in der üblichen Weise zurückgewonnen und direkt, ggf. auch nach weiterer Reinigung wieder eingesetzt werden.

Ein solches Verfahren wird vorzugsweise säurekatalysiert durchgeführt. Als Katalysatoren verwendet werden geeignete Lewissäuren oder Brönstedt-Säuren.

Bevorzugte Katalysatoren sind HCl, H₂SO₄, *p-*Toluolsulfonsäure, CF₃SO₃H als Brönstedt-Säuren und ZnCl₂, CuSO₄, FeCl₃ AlCl₃, MgCl₂ und MgBr₂ als Lewissäuren. Die Katalysatoren können nach Beendigung der Reaktion in der üblichen Weise zurückgewonnen und direkt, ggf. nach Reinigung wieder eingesetzt werden.

Es ist auch möglich, dass man anstelle der Carbonylverbindung R¹R²C=O deren Dimethyl- oder Diethylacetal einsetzt. Der dabei anfallende Methanol oder Ethanol kann aus dem Reaktionsgemisch zurückgewonnen werden, vorzugsweise destillativ.

Auch ist es vorteilhaft das bei direktem Einsatz der Carbonylverbindung R¹R¹C=O während der Kondensationsreaktion entstehende Wasser aus dem Reaktionsgemisch zu entfernen. Durch die Entfernung von entstehendem Wasser bzw. Alkohol aus der Reaktionsmischung werden ein höherer Umsatz und eine größere Selektivität an gewünschtem Kondensationsprodukt erzielt. Zur Wasser-/Alkohlentfernung können auch zusätzlich noch Schleppmittel wie z.B. Toluol verwendet werden, so dass Wasser bzw. Alkohole in Form von Azeotropen destillativ entfernt werden können.

Gegenstand der Erfindung ist auch ein Verfahren zur Herstellung von Verbindungen der Formel **II,** dadurch gekennzeichnet dass man eine Verbindung der allgemeinen Formel **III** 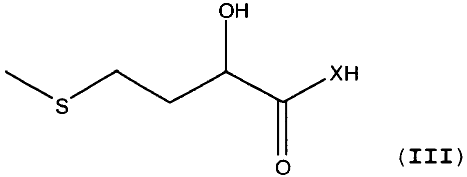 mit einem Kohlensäurederivat X¹X²C=O umsetzt, wobei X¹ und X² gleich oder verschieden sind und unabhängig voneinander Chlor, OCCl₃ OCH₃, OCH₂CH₃ oder über den Stickstoff gebundenes Imidazolyl bzw. Triazolyl sein können.

Da Phosgen (X¹, X² =Cl) als Reagenz problematisch ist, wird bevorzugt das gut handhabbare Diphosgen (X¹ - Cl, X² = OCCl₃) als reaktives Kohlensäureäquivalent verwendet. Gut geeignet und gut handhabbar sind aber auch Dimethylcarbonat oder Diethylcarbonat und die gezeigten N-haltigen Kohlensäureäquivalente wie z.B. Carbonyldiimidazol.

Eine solche Reaktion kann vorteilhafterweise sowohl sauer oder basisch katalysiert durchgeführt werden. Als saure Katalysatoren können die oben bereits genannten Brönstedt- oder Lewissäuren eingesetzt werden(??). Als basische Katalysatoren eignen sich insbesondere Alkalimetallalkoholate von C₁-C₄-Alkoholen wie beispielsweise Natriummethoxid oder -ethoxid oder auch Kalium-tert-butylat.

Eine weitere geeignete Verfahrensvariante zur Herstellung von Verbindungen der Formel **I** mit X = NH, dadurch gekennzeichnet, dass man 2-Hydroxy-4-methylthiobuttersäurenitril der Formel **IV** 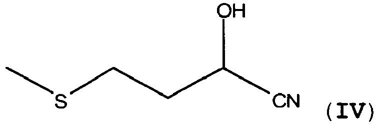 mit einer Carbonylverbindung R¹R²C=O in Gegenwart von Säure und einem Carbonsäureanhydrid umsetzt, wobei R¹ und R² die oben angegebene Bedeutung haben. Dies hat den Vorteil, dass man sich die Vorstufe des 2-Hydroxy-4-methylthiobuttersäure -amids (MHA-Amid) einspart.

Bei einer solchen Verfahrensvariante werden als Säure bevorzugt Schwefelsäure und/oder Essigsäure und als Carbonsäureanhydrid vorzugsweise Essigsäureanhydrid verwendet.

Alle Verfahrensvarianten haben den Vorteil, dass sie in einfacher Weise und mit z.T. guten bis sehr guten Ausbeuten durchgeführt werden können.

Die nachfolgenden Beispiele dienen der näheren Erläuterung der Erfindung ohne beschränkend zu sein.

### Beispiele:

### Beispiel 1:

Darstellung von 5-(2-(methylthio)ethyl)-1,3-dioxolan-4-on (**2**) aus 2-Hydroxy-4-(methylthio)butansäure Calciumsalz (**1**) und Formalinlösung durch Brönstedt-Säurekatalyse im Zweiphasengemisch:

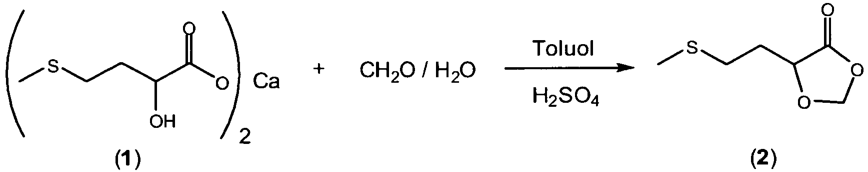

10.0 g (29.5 mmol) 2-Hydroxy-4-(methylthio)butansäure Calciumsalz (**1**) wurden in einem 500 mL-Dreihalsrundkolben in 150 mL Wasser und 150 mL Toluol vorgelegt und mit 3.5 g (34.6 mmol) 97%iger Schwefelsäure versetzt. Nach Zugabe von 50 g (0.58 mol, 19.6 eq.) 37%iger Formalinlösung wurde auf Siedetemperatur erhitzt und 16 h lang bei dieser Temperatur gerührt. Nach dem Abkühlen wurden die Phasen getrennt und die wässrige Phase zweimal mit je 50 mL Toluol gewaschen. Die vereinigten org. Phasen wurden einmal mit 50 mL NaCl-Lsg. gewaschen, über M_{g}SO₄ getrocknet und am Rotationsverdampfer eingeengt. Das erhaltene Rohprodukt wurde anschließend destilliert (Sdp. = 125°C/1.5 mbar) Man erhielt 7.7 g (47.6 mmol, Ausbeute = 81 %) 5-(2-(methylthio)ethyl)-1,3-dioxolan-4-on (**2**) als farblose Flüssigkeit.

¹H-NMR von 5-(2-(methylthio)ethyl)-1,3-dioxolan-4-on (**2**) (500 MHz, CDCl₃): δ = 2.02-2.21 (m, 2H, CH₂); 2.12 (s, 3H, SCH₃); 2.62-2.72 (m, 2H, SCH₂): 4.39 - 4.41 (m, 1H, CH) ; 5.44 (s, 1H, CH); 5.55 (s, 1H, CH)

¹³C-NMR von 5-(2-(methylthio)ethyl)-1,3-dioxolan-4-on (**2**) (125.8 MHz, CDCl₃) : δ = 15.29 (SCH₃) ; 29.38 (SCH₂) ; 29.74 (CH₂); 71.49 (CH); 94.26 (OCH₂O) ; 172.80 (C=O)

| Elementaranalyse für C₆H₁₀O₃S (M = 162.21 g/mol): | |
|---|---|
| Berechnet: | C 44.43; H 6.21; S 19.77 |
| Gefunden: | C 44.22; H 6.36; S 19.69 |

### Beispiel 2:

Darstellung von 5-(2-(methylthio)ethyl)-1,3-dioxolan-4-on (**2**) aus 2-Hydroxy-4-(methylthio)butansäure (3) und Trioxan oder Paraformaldehyd durch Brönstedt-Säurekatalyse:

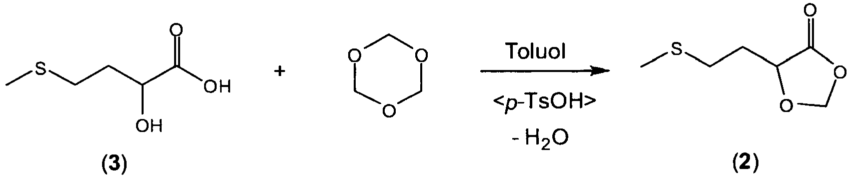

5.0 g (33.3 mmol) 2-Hydroxy-4-(methylthio)butansäure (**3**) und 5.0 g (55.5 mmol, 1,67 eq.) 1,3,5-Trioxan (alternativ 5.0 g Paraformaldehyd) wurden in einem 100 mL-Dreihalsrundkolben in 50 mL Toluol vorgelegt, mit einer Spatelspitze p-Toluolsulfonsäure versetzt und zum Sieden erhitzt. Nach 12 h wurde das Lösungsmittel am Rotationsverdampfer abdestilliert und das erhaltene Rohprodukt im Vakuum destilliert. Man erhielt 4.6 g (28.5 mmol, Ausbeute = 86 %) 5-(2-(methylthio)ethyl)-1,3-dioxolan-4-on (**2**) als farblose Flüssigkeit. Die NMR-Daten stimmten mit denen aus Beispiel 1 überein.

### Beispiel 3:

Darstellung von 2,2-Dimethyl-5-(2-(methylthio)ethyl)-1,3-dioxolan-4-on (**4**) aus 2-Hydroxy-4-(methylthio)butansäure (**3**) und Aceton durch Brönstedt-Säurekatalyse:

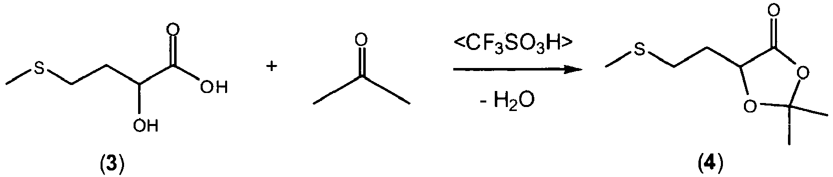

5.0 g (33.3 mmol) 2-Hydroxy-4-(methylthio)butansäure (**3**) wurden in einem 250 mL-Dreihalsrundkolben in 100 mL Aceton vorgelegt, mit wenigen Tropfen Trifluormethansulfonsäure oder Schwefelsäure versetzt und 16 h lang bei RT gerührt. Anschließend wurde die Reaktionsmischung am Rotationsverdampfer eingeengt, in 100 mL Diethylether aufgenommen und zweimal mit je 25 mL ges. NaCl-Lsg. extrahiert. Die Etherphase wurde über MgSO₄ getrocknet, am Rotationsverdampfer eingeengt und das erhaltene Rohprodukt anschließend im Vakuum über eine Vigreuxkolonne destilliert (Sdp. = 122°C/1 mbar). Man erhielt 5.2 g (27.4 mmol, Ausbeute = 82 %) 2,2-Dimethyl-5-(2-(methylthio)ethyl)-1,3-dioxolan-4-on (**4**) als farbloses Öl.

¹H-NMR von 2,2-Dimethyl-5-(2-(methylthio)ethyl)-1,3-dioxolan-4-on (**4**) (500 MHz, CDCl₃) : δ = 1.55 (s, 3H, CH₃) ; 1.61 (s, 3H, CH₃); 1.95-2.20 (m, 2H, CH₂); 2.11 (s, 3H, SCH); 2.62-2.66 (m, 2H, SCH₂); 4.55 (dd, ³J = 7.5 Hz, ²J = 4.4 Hz, 1H, CH)

¹³C-NMR von 2,2-Dimethyl-5-(2-(methylthio)ethyl)-1,3-dioxolan-4-on (**4**) (125.8 MHz, CDCl₃): δ = 14.96 (SCH₃); 25.46 (CH₃); 26.92 (CH₃); 29.04 (CH₂); 30.73 (CH₂); 72.18 (CH); 110.37 (C); 172.68 (C=O)

| Elementaranalyse für C₈H₁₄O₃S (M = 190.26 g/mol): | |
|---|---|
| Berechnet: | C 50.50; H 7.42; S 16.85 |
| Gefunden: | C 50.28; H 7.63; S 16.88 |

### Beispiel 4:

Darstellung von 2,2-Dimethyl-5-(2-(methylthio)ethyl)-1,3-dioxolan-4-on (**4**) aus 2-Hydroxy-4-(methylthio)butansäure (**3**) und Aceton durch Lewis-Säurekatalyse:

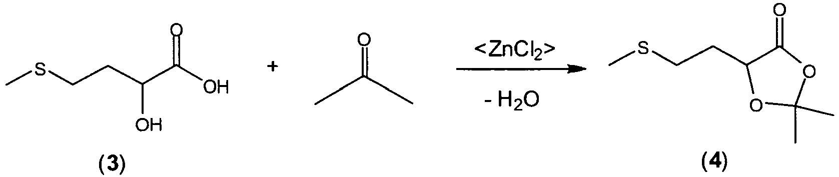

1.0 g (6.7 mmol) 2-Hydroxy-4-(methylthio)butansäure (**3**) wurden in einem 100 mL-Dreihalsrundkolben in 20 mL Aceton vorgelegt, mit 1.0 eq. Lewis-Säure (893 mg ZnCl₂ alternativ 1.69 g MgBr₂ x 2 Et₂O oder 1.38 g BF₃ x 2 H₂O) versetzt und 16 h lang bei RT gerührt. Anschließend wurde die Reaktionsmischung am Rotationsverdampfer eingeengt, in 100 mL Diethylether aufgenommen, mit 50 mL Wasser und zweimal mit je 25 mL ges. NaCl-Lsg. gewaschen. Die Etherphase wurde dann über MgSO₄ getrocknet, am Rotationsverdampfer eingeengt und das erhaltene Rohprodukt anschließend im Vakuum am Kugelrohr destilliert. Man erhielt 1.1 g (5.8 mmol, Ausbeute = 87 %) 2,2-Dimethyl-5-(2-(methylthio)ethyl)-1,3-dioxolan-4-on (**4**) als farbloses Öl. Die NMR-Daten stimmten mit denen aus Beispiel 3 überein.

### Beispiel 5:

Darstellung von 2,2-Dimethyl-5-(2-(methylthio)ethyl)-1,3-dioxolan-4-on (**4**) aus 2-Hydroxy-4-(methylthio)butansäure (**3**) und Aceton durch Umketalisierung:

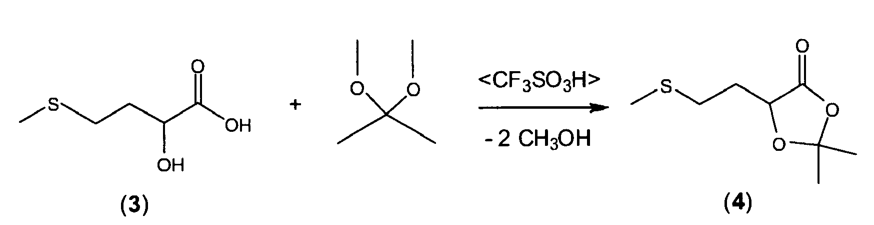

5.0 g (33.3 mmol) 2-Hydroxy-4-(methylthio)butansäure (**3**) und 5.0 g (48.0 mmol, 1.44 eq.) 2,2-Dimethoxypropan wurden in einem 100 mL-Dreihalsrundkolben in 50 mL Tetrahydrofuran vorgelegt und zum Sieden erhitzt. Nach 3 h wurde das Lösungsmittel am Rotationsverdampfer abdestilliert und das erhaltene Rohprodukt anschließend im Vakuum destilliert. Man erhielt 5.6 g (29.7 mmol, Ausbeute = 89 %) 2,2-Dimethyl-5-(2-(methylthio)ethyl)-1,3-dioxolan-4-on (**4**) als farbloses Öl. Die NMR-Daten stimmten mit denen aus Beispiel 3 überein.

### Beispiel 6:

Darstellung von 2-Ethyl-2-methyl-5-(2-(methylthio)ethyl)-1,3-dioxolan-4-on (**5**) aus 2-Hydroxy-4-(methylthio)butansäure (**3**) und Ethylmethylketon:

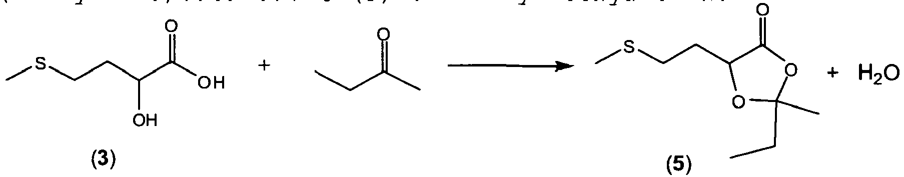

35.0 g (205 mmol) 88%ige 2-Hydroxy-4-(methylthio)butansäure (**3**) wurden in 350 mL Ethylmethylketon gegeben und unter Rückfluss für 5 h gehalten. Nach dem Abkühlen wurde das Lösungsmittel mit dem entstandenen Wasser am Rotationsverdampfer abgezogen und der Rückstand im Vakuum destilliert (Sdp = 103°C, 0.4 mbar). Man erhielt 26.5 g (mmol, Ausbeute = 56 %) 2-Ethyl-2-methyl-5-(2-(methylthio)ethyl)-1,3-dioxolan-4-on (**5**) als farblose Flüssigkeit. Das abgezogene Ethylmethylketon wurde über MgSO₄ getrocknet und konnte anschließend wieder für die nächste Umsetzung verwendet werden.

¹H-NMR von 2-Ethyl-2-methyl-5-(2-(methylthio)ethyl)-1,3-dioxolan-4-on (**5**) (Diastereomerengemisch, Verhältnis 63:37) (500 MHz, CDCl₃) : δ = 0.96-1.00 (m, 3H, CH₃); 1.52, 1.56 (s, 3H, CH₃); 1.80-1.88 (m, 2H, CH₂); 1.97-2.18 (m, 2H, CH₂); 2.11 (s, 3H, SCH₃); 2.63-2.67 (m, 2H, CH₂); 4.54-4.58 (m, 1H, CH)

¹³C-NMR von 2-Ethyl-2-methyl-5-(2-(methylthio)ethyl)-1,3-dioxolan-4-on (**5**) (Diastereomerengemisch, Verhältnis 63:37) (125.8 MHz, CDCl₃) : δ = 7.23, 7. 87 (CH₃); 15.24 (SCH₃); 23.86, 25.09 (CH₂); 29.34, 29.51, 30.97, 31.54, 32.42, 32.60 (CH₃, 2 x CH₂) ; 72.25, 73.00 (CH); 112.29, 112.83 (C); 173.04, 173.09 (C=O)

| Elementaranalyse für C₉H₁₆O₃S (M = 204.29 g/mol): | |
|---|---|
| Berechnet: | C 52.91; H 7.89; S 15.70 |
| Gefunden: | C 53.04; H 8.02; S 15.46 |

Beispiel 7:

Darstellung von 2-tert-Butyl-5-(2-(methylthio)ethyl)-1,3-dioxolan-4-on (**6**) 2-Hydroxy-4-(methylthio)butansäure Calciumsalz (**1**) und Pivalaldehyd unter Brönstedt-Säurekatalyse:

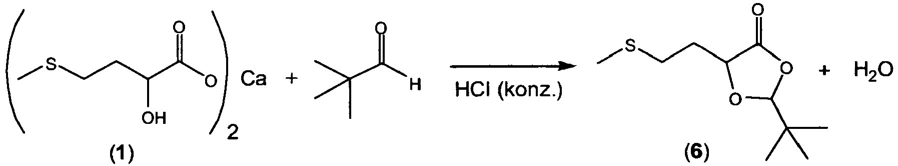

6.77 g (20 mmol) 2-Hydroxy-4-(methylthio)butansäure Calciumsalz (**1**) wurden unter Rühren und Eiskühlung langsam mit 13.8 g konz. Salzsäure versetzt. Es bildete sich eine klare Lösung. Anschließend wurden unter Schutzgasatmosphäre 15 mL Toluol und 3.45 g (40 mmol) frisch destillierter Pivalaldehyd zugegeben auf 75°C erwärmt, wobei das Zweiphasengemisch klar wurde. Dann wurde 7 h lang bei dieser Temperatur gerührt. Nach dem Abkühlen bildeten sich zwei Phasen aus. Die organische Toluolphasen wurde abgetrennt und die wässrige Phase zweimal mit je 10 mL Toluol gewaschen. Die vereinigten org. Phasen wurden dreimal mit je 15 mL Wasser gewaschen, über Na₂SO₄ getrocknet und nach Filtration am Rotationsverdampfer eingeengt. Das erhaltene Rohprodukt wurde abschließend im Hochvakuum von letzten Lösungsmittelresten befreit. Man erhielt 2.62 g (12.0 mmol, Ausbeute = 30 %) 2-*tert*-Butyl-5-(2-(methylthio)ethyl)-1,3-dioxolan-4-on (**6**) als leicht gelbliches Öl.

¹H-NMR von 2-*tert*-Butyl-5-(2-(methylthio)ethyl)-1,3-dioxolan-4-on (**6**) (Diastereomerengemisch) (500 MHz, CDCl₃): δ = 0.96, 0.98 (s, 9H, CH₃); 1.99-2.22 (m, 2H, CH₂); 2.09 (s, 3H, SCH₃); 2.64-2.69 (m, 2H, CH₂); 4.43-4.46, 4.51-4.54 (m, 1H, CH); 5.15, 5.28 (s, 1H, CH)

¹³C-NMR von 2-*tert*-Butyl-5-(2-(methylthio)ethyl)-1,3-dioxolan-4-on (**6**) (Diastereomerengemisch) (125.8 MHz, CDCl₃) : δ = 15.21, 15.25 (SCH₃) ; 23.24, 23.44 (CH₃) ; 29.37, 29.39, 30.22, 30.33, 34.24, 35.74 (C, 2 x CH₂); 73.15, 73.42 (CH); 109.43, 110.46 (CH); 173.17, 173.27 (C=O)

| Elementaranalyse für C₁₀H₁₈O₃S (M = 218.31 g/mol) : | |
|---|---|
| Berechnet: | C 55.01; H 8.33; S 14.69 |
| Gefunden: | C 55.36; H 8.52; S 14.23 |

### Beispiel 8:

Darstellung von 3-(2-(Methylthio)ethyl)-1,4-dioxaspiro[4.5]decan-2-on (**7**) aus 2-Hydroxy-4-(methylthio)butansäure (**3**) und Cyclohexanon:

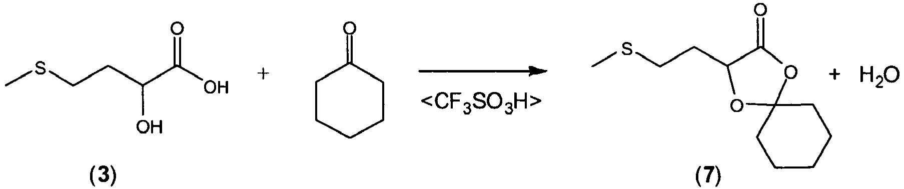

10.0 g (66.6 mmol) 2-Hydroxy-4-(methylthio)butansäure (**3**) und 13.1 g (133.2 mmol, 2.0 eq.) Cyclohexanon wurden in einem 250 mL-Dreihalsrundkolben in 100 mL THF vorgelegt, mit wenigen Tropfen Trifluormethansulfonsäure versetzt und 16 h lang bei RT gerührt. Anschließend wurde die Reaktionsmischung am Rotationsverdampfer eingeengt. Der erhaltene Rückstand wurde mit 100 mL einer Mischung aus 10 mL Dichlormethan und 90 mL n-Hexan aufgelöst und zweimal mit je 50 mL Wasser und einmal mit 50 mL ges. NaCl-Lsg. gewaschen. Die org. Phase wurde anschließend über MgSO₄ getrocknet und am Rotationsverdampfer eingeengt. Das erhaltene Rohprodukt wurde dann über eine Kieselgelsäule mit n-Hexan/Ethylacetat=15:1 chromatographiert. Nach dem Einengen am Rotationsverdampfer wurden die letzten Lösungsmittelreste im Hochvakuum entfernt. Man erhielt 11.2 g (48.6 mmol, Ausbeute = 73 %) 3-(2-(Methylthio)ethyl)-1,4-dioxaspiro[4.5]decan-2-on (**7**) als farblose Flüssigkeit.

¹H-NMR von 3-(2-(Methylthio)ethyl)-1,4-dioxaspiro[4.5]decan-2-on (**7**) (500 MHz, CDCl₃) : δ = 1.38-1.88 (m, 10H, 5 x CH₂); 1.96-2.20 (m, 2H, CH₂); 2.11 (s, 3H, SCH₃); 2.65 (t, ³J = 7.4 Hz, 2H, SCH₂); 4.55 (dd, ³J = 7.6, ²J = 4.5 Hz, 1H, CH)

¹³C-NMR von 3-(2-(Methylthio)ethyl)-1,4-dioxaspiro[4.5]decan-2-on (**7**) (125.8 MHz, CDCl₃) δ = 22.86 (SCH₃) ; 23.00 (CH₂); 24.50 (CH₂) ; 29.38 (SCH₂); 31.13 (CH₂); 35.24 (CH₂) ; 36.77 (CH) ; 72.25 (CH); 111.49 (C); 173.07 (C=O)

### Beispiel 9:

Darstellung von 5-(2-(Methylthio)ethyl)-1,3-dioxolan-2,4-dion (**8**) aus 2-Hydroxy-4-(methylthio)butansäure (**3**) und Diphosgen:

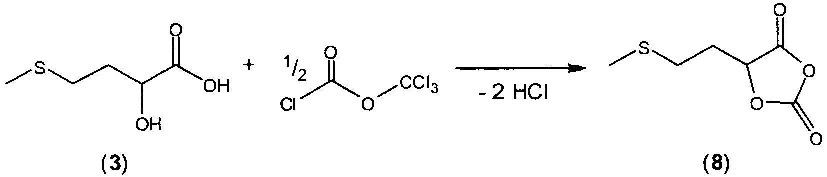

1.5 g (10.0 mmol) 2-Hydroxy-4-(methylthio)butansäure (**3**) wurden in einem 50 mL Schlenkkolben in 10 mL trockenem THF vorgelegt und unter Argon-Atmosphäre 1.5 mL (12.0 mmol) Diphosgen über einen Zeitraum von 15 Min. zugegeben. Nach Zugabe von 30 mg Aktivkohle wurde das Reaktionsgemisch 12 h lang bei RT gerührt. Anschließend wurde die Reaktionslösung über ein Celitbett filtriert, bei Raumtemperatur am Rotationsverdampfer eingeengt und 4 h lang im Hochvakuum getrocknet. Man erhielt 1.1 g (9.7 mmol, Ausbeute = 97 %) 5-(2-(Methylthio)ethyl)-1,3-dioxolan-2,4-dion (**8**) als gelbliches Öl.

¹H-NMR von 5-(2-(Methylthio)ethyl)-1,3-dioxolan-2,4-dion (**8**) (500 MHz, CDCl₃) : δ = 2. 10 (s, 3H, SCH₃); 2.20-2.40 (m, 2H, CH₂); 2.60-2.80 (m, 2H, SCH₂); 5.20-5.30 (m, 1H, CH)

### Beispiel 10:

Darstellung von 2,2-Dimethyl-5-(2-(methylthio)ethyl)oxazolidin-4-on (**10**) aus 2-Hydroxy-4-(methylthio)butansäureamid (**9**) und Aceton durch Brönstedt-Säurekatalyse:

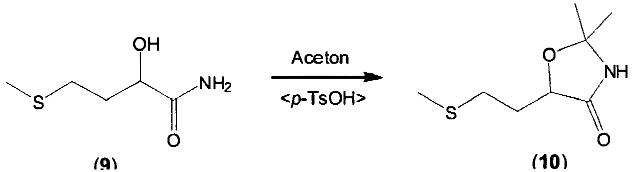

In einem 250 mL Dreihalskolben mit Wasserabscheider und Rückflusskühler wurden 14.9 g (0.1 mol) 2-Hydroxy-4-(methylthio)butansäureamid (**9**) in 150 mL Toluol vorgelegt, mit 11.6 g Aceton (0.2 mol) und 0.8 g p-Toluolsulfonsäure versetzt und unter Rühren langsam auf Siedetemperatur erhitzt. Dabei klärte sich die trübe Suspension bei 90°C auf. Die gesamte Lösung wurde 14 h am Rückfluss gekocht. Dabei wurde insgesamt zweimal die überdestillierte Toluolphase abgelassen und anschließend zweimal jeweils mit 11.6 g Aceton ergänzt. Nach Abkühlung wurde die trübe Reaktionslösung filtriert und das Filtrat einmal mit 100 mL verdünnter NaH-CO₃-Lösung, zweimal mit je 100 mL H₂O und abschließend einmal mit 100 mL gesättigter Natriumchlorid-Lösung gewaschen. Danach wurde die Toluolphase über Na₂SO₄ getrocknet. Nach der Filtration wurde das Lösungsmittel am Rotationsverdampfer im Vakuum abgezogen. Man erhielt 13.2 g eines orangebraunen Öls, das langsam kristallisierte. Zur Umkristallisation wurden 30 mL n-Hexan zugegeben, kurz auf Siedetemperatur erhitzt, anschließend auf RT abgekühlt und über Nacht stehen gelassen. Am nächsten Tag wurde der auskristallisierte Feststoff abfiltriert und im Hochvakuum getrocknet. Man erhielt 11.5 g (0.06 mol, M = 189.28 g/mol, Ausbeute = 60 %) 2,2-Dimethyl-5-(2-(methylthio)ethyl)-oxazolidin-4-on (**8**) als leicht gelblichen Feststoff (Smp. = 84°C).

¹H-NMR von 2,2-Dimethyl-5-(2-(methylthio)ethyl)-4-oxazolidinon (**10**) (500 MHz, DMSO-d6): δ = 1.34 (s, 3H, CH₃); 1.36 (s, 3H, CH₃); 1.73-1.78 (m, 1H, CH); 1.87-1.92 (m, 1H, CH); 2.04 (s, 3H, SCH₃); 2.43-2.56 (m, 2H, CH₂); 4.23-4.28 (m, 1H, CH); 8.83 (bs, 1H, NH).

¹³C-NMR von 2,2-Dimethyl-5-(2-(methylthio)ethyl)-4-oxazolidinon (**10**) (125.8 MHz, DMSO-d6): δ = 14.50 (SCH₃) ; 28.07, 28.70, 29. 10, 31.66 (2 x CH₂, 2 x CH₃) ; 74.53 (CH); 89.60 (C); 171.94 (C=O).

| Elementaranalyse für C₈H₁₅NO₂S (M = 189.28 g/mol): | |
|---|---|
| Berechnet: | C 50.76; H 7.99; N 7.40; S 16.94 |
| Gefunden: | C 50.90; H 8.11; N 7.31; S 16.90 |

### Beispiel 11:

Darstellung von 2,2-Dimethyl-5-(2-(methylthio)ethyl)-oxazolidin-4-on (**10**) aus 2-Hydroxy-4-(methylthio)butansäureamid (**9**) durch Umketalisierung:

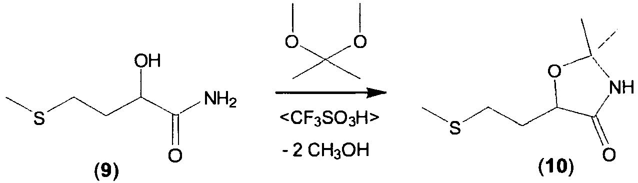

10.0 g (67.0 mmol) 2-Hydroxy-4-(methylthio)butansäureamid (**9**) wurden in einem 250 mL-Dreihalskolben in 70 mL trockenem Tetrahydrofuran suspendiert und mit 13.96 g (134.0 mmol, 2.0 eq.) Dimethoxypropan versetzt. Nach Zugabe weniger Tropfen Trifluormethansulfonsäure wurde das Reaktionsgemisch 16 h lang bei Raumtemperatur gerührt. Anschließend wurde das Lösungsmittel am Rotationsverdampfer bei 100 mbar/30°C entfernt. Der ölige Rückstand wurde in 100 mL Diethylether gelöst und zweimal mit je 50 mL Wasser gewaschen. Die Etherphase wurde über M₉SO₄ getrocknet und am Rotationsverdampfer eingeengt. Der erhaltene Feststoff wurde anschließend aus 100 mL *n*-Hexan umkristallisiert, abfiltriert und letzte Lösungsmittelreste im Hochvakuum entfernt. Man erhielt 11.8 g (62 mmol, Ausbeute = 93 %) 2,2-Dimethyl-5-(2-(methylthio)ethyl)oxazolidin-4-on (**10**) als farblosen Feststoff. Die NMR-Daten stimmten mit denen aus Beispiel 10 überein.

| Elementaranalyse für C₈H₁₅NO₂S (M = 189.28 g/mol) : | |
|---|---|
| Berechnet: | C 50.76; H 7.99; N 7.40; S 16.94 |
| Gefunden: | C 50.96; H 8.14; N 7.31; S 16.88 |

### Beispiel 12:

Darstellung von 2,2-Dimethyl-5-(2-(methylthio)ethyl)-oxazolidin-4-on (**10**) aus 1-Hydroxy-3-(methylthio)propancarbonitril (**11**):

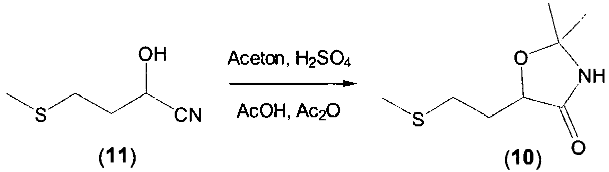

In einem 100 mL Dreihalskolben wurden 13.1 g 96%iges 1-Hydroxy-3-(methylthio)propancarbonitril (**11**) (0.1 mol) und 7.0 g Aceton (0.12 mol) in 30 mL Eisessig bei 10°C gelöst. Dann wurden 5 mL Acetanhydrid (0.05 mol) langsam zugetropft. Anschließend wurde eine Mischung aus 10 mL konz. Schwefelsäure und 10 mL Eisessig bei 0°C langsam zugegeben. Dabei musste darauf geachtet werden, dass die gesamte Reaktionslösung nicht wärmer als 0°C wurde. Man erhielt eine zähe, gelbliche, kaum rührbare Suspension. Nach der vollständigen Zugabe wurde 1 h lang bei 10°C und abschließend 15 min bei RT gerührt. Die Reaktionslösung wurde auf Eis (ca. 150 g) gegossen und danach dreimal mit je 100 mL Diethylether extrahiert. Die Etherphase wurde einmal mit gesättigter Natriumhydrogencarbonatlösung und anschließend mit gesättigter Kochsalzlösung gewaschen und über Natriumsulfat getrocknet. Das Na₂SO₄ wurde abfiltriert und der Ether im Vakuum abgezogen. Man erhielt 4.5 g eines orangebraunen Öls, welches aus n-Hexan umkristallisiert wurde. Nach Filtration und Entfernung letzter Lösungsmittelreste im Hochvakuum konnten 2.8 g (14.8 mmol, M = 189.28 g/mol, Ausbeute = 15 %) 2,2-Dimethyl-5-(2-(methylthio)ethyl)-4-oxazolidinon (**10**) als leicht gelblicher Feststoffs isoliert werden. Die NMR-Daten stimmten mit denen aus Beispiel 10 überein.

### Beispiel 13:

Darstellung von 5-(2-(Methylthio)ethyl)-2-phenyloxazolidin-4-on (**12**) aus 2-Hydroxy-4-(methylthio)butansäureamid (**9**) durch Brönstedt-Säurekatalyse: 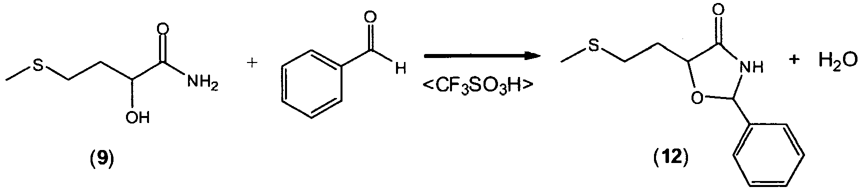 5.0 g (33.5 mmol) 2-Hydroxy-4-(methylthio)butansäureamid (**9**) wurden in einem 100 mL-Dreihalskolben in 35 mL trockenem Tetrahydrofuran suspendiert und mit 7.1 g (67 mmol, 2.0 eq.) frisch destillierten Benzaldehyd versetzt. Nach Zugabe von wenigen Tropfen Trifluormethansulfonsäure wurde das Reaktionsgemisch 16 h lang bei Raumtemperatur gerührt. Die klare Reaktionslösung wurde am Rotationsverdampfer eingeengt und der erhaltene Rückstand in 100 mL Diethylether aufgenommen. Anschließend wurde dreimal mit je 30 mL Wasser und einmal mit 30 mL ges. NaCl-Lsg. gewaschen. Die Etherphase wurde über MgSO₄ getrocknet und am Rotationsverdampfer eingeengt. Das erhaltene Produktgemisch wurde dann über eine fraktionierte Kristallisation aufgetrennt. Aus 100 mL Dichlormethan/Diethylether=1:1 wurde insgesamt 2.8 g eines Feststoffs isoliert, der anschließend aus Diethylether umkristallisiert wurde. Man erhielt 2.1 g (8.8 mmol, Ausbeute = 26 %) 5-(2-(Methylthio)ethyl)-2-phenyloxazolidin-4-on (**12**) als farblosen Feststoff (Smp. = 130°C).

¹H-NMR von 5-(2-(Methylthio)ethyl)-2-phenyloxazolidin-4-on (**12**) (500 MHz, CDCl₃): δ = 2.00-2.30 (m, 2H, CH₂); 2.11 (s, 3H, SCH₃) ; 2.63-2.75 (m, 2H, SCH₂) ; 4.49-4.53 (m, 1H, CH) ; 6.03-6.05 (m, 1H, CH); 6.24 (bs, 1H, NH); 7.40 - 7.50 (m, 5H, H_{Phenyl})

¹³C-NMR von 5-(2-(Methylthio)ethyl)-2-phenyloxazolidin-4-on (**12**) (125.8 MHz, CDCl₃) : δ = 19.67 (SCH₃); 29.93 (SCH₂); 31.74 (CH₂); 76.68 (CH); 87.33 (CH); 127.23, 129.28, 130.49, 138, 12 (C_{Phenyl}); 175.25 (C=O)

| Elementaranalyse für C₁₂H₁₅NO₂S (M = 237.32 g/mol): | |
|---|---|
| Berechnet: | C 60.73; H 6.37; N 5.90; S 13.51 |
| Gefunden: | C 60.61; H 6.27; N 5.66; S 13.49 |

### Beispiel 14:

Darstellung von 2-Ethyl-2-methyl-5-(2-(methylthio)ethyl)oxazolidin-4-on (**13**) aus 2-Hydroxy-4-(methylthio)butansäureamid (**9**) durch Brönstedt-Säurekatalyse:

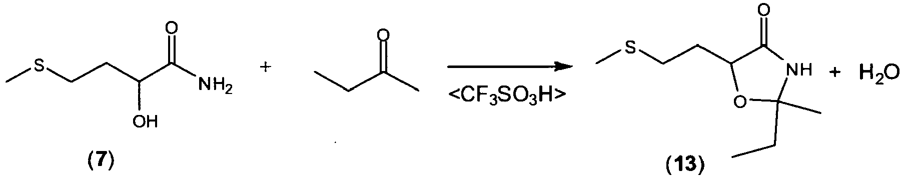

5.0 g (33.5 mmol) 2-Hydroxy-4-(methylthio)butansäureamid (**9**) wurden in einem 100 mL-Dreihalskolben in 35 mL trockenem Tetrahydrofuran suspendiert und mit 4.8 g (67 mmol, 2.0 eq.) Ethylmethylketon versetzt. Nach Zugabe von wenigen Tropfen Trifluormethansulfonsäure wurde das Reaktionsgemisch 5 Tage bei Raumtemperatur gerührt. Die klare Reaktionslösung wurde am Rotationsverdampfer eingeengt, der erhaltene Rückstand in 100 mL Diethylether aufgenommen und dreimal mit je 30 mL Wasser und einmal mit 30 mL ges. NaCl-Lsg. gewaschen. Die vereinten Etherphasen wurden über MgSO₄ getrocknet, am Rotationsverdampfer eingeengt und der Rückstand zweimal aus einem Diethylether/n-Hexan-Gemisch umkristallisiert. Man erhielt 5.1 g (24.9 mmol, Ausbeute = 74 %) 2-Ethyl-2-methyl-5-(2-(methylthio)ethyl)oxazolidin-4-on (**13**) als farblosen Feststoff (Smp. = 62°C).

¹H-NMR von 2-Ethyl-2-methyl-5-(2-(methylthio)ethyl)oxazolidin-4-on (**13**) (Diastereomerengemisch) (500 MHz, CDCl₃) : δ = 0. 95 (t, ³J = 7.4 Hz, 3H, CH₃); 1.44 - 1.46 (pd, 3H, CH₃); 1.62-1.80 (m, 2H, CH₂); 1.90-2.16 (m, 2H, CH₂); 2. 12 (s, 3H, SCH₃); 2.60-2.70 (m, 2H, SCH₂); 4.42-4.48 (m, 1H, CH); 6.60-6.80 (bd, 1H, NH)

¹³C-NMR von 2-Ethyl-2-methyl-5-(2-(methylthio)ethyl)oxazolidin-4-on (**13**) (Diastereomerengemisch) (125.8 MHz, CDCl3) : δ = 7.82, 7.97 (CH₃); 15.36, 15.37 (SCH₃); 26.55, 27.62, 29.55, 29.78, 31.79, 32.42, 34.09, 34.51 (3 x CH₂, 1 x CH₃); 75.18, 76.27 (CH); 92.56, 92.81 (C); 174.13, 174.19 (C=O)

| Elementaranalyse für C₉H₁₇NO₂S (M = 203.30 g/mol): | |
|---|---|
| Berechnet: | C 53.17; H 8.43; N 6.89; S 15.77 |
| Gefunden: | C 52.46; H 8.27; N 6.49; S 15.71 |

### Beispiel 15:

Darstellung von 2-(2-(Methylthio)ethyl)-1-oxa-4-azaspiro[4.5]decan-3-on (**14**) aus 2-Hydroxy-4-(methylthio)butansäureamid (**9**) durch Brönstedt-Säurekatalyse:

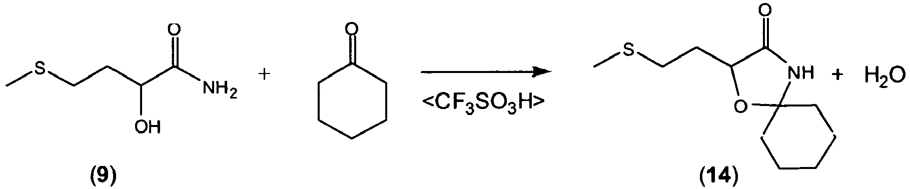

10.0 g (67.0 mmol) 1-Hydroxy-3-(methylmercapto)-butansäureamid (**9**) wurden in einem 250 mL-Dreihalskolben in 150 mL trockenem Toluol suspendiert und mit 32.9 g (336 mmol, 5.0 eq.) Cyclohexanon versetzt. Nach Zugabe weniger Tropfen Trifluormethansulfonsäure wurde das Reaktionsgemisch zum Sieden erhitzt und 1 h lang bei dieser Temperatur gerührt. Die Reaktionslösung wurde anschließend abgekühlt und zweimal mit je 50 mL Wasser extrahiert. Die org. Phase wurde über M₉SO₄ getrocknet und am Rotationsverdampfer bei 70 mbar/40°C entfernt. Der erhaltene Feststoff wurde aus n-Hexan/EtOAc umkristallisiert, abfiltriert, getrocknet und letzte Lösungsmittelreste im Hochvakuum entfernt. Man erhielt 12.4 g (54 mmol, Ausbeute = 80 %) 2-(2-(Methylthio)ethyl)-1-oxa-4-azaspiro[4.5]decan-3-on (**14**) als farblosen Feststoff (Smp. = 109°C).

¹H-NMR von 2-(2-(Methylthio)ethyl)-1-oxa-4-azaspiro[4.5]decan-3-on (**14**) (500 MHz, CDCl₃): δ = 1.38-1.80 (m, 10H, 5 x CH₂); 1.90-2.18 (m, 2H, CH₂); 2.12 (s, 3H, SCH₃) ; 2.64 (t, ³*J* = 7.6 Hz, 2H, CH₂) ; 4.44 (dd, ³*J* = 7.6 Hz, ²*J* = 2.1 Hz, 1H, CH); 8.41 (bs, 1H, NH)

¹³C-NMR von 2-(2-(Methylthio)ethyl)-1-oxa-4-azaspiro[4.5]decan-3-on (**14**) (125.8 MHz, CDCl₃): δ = 15.42 (SCH₃); 23.02, 23.12, 24. 68 (3 x CH₂); 29.52 (SCH₂); 32.30 (CH₂); 37.85, 38.93 (2 x CH₂); 75.33 (CH); 91.98 (C); 174.54 (C=O)

| Elementaranalyse für C₁₁H₁₉NO₂S (M = 229.34 g/mol) : | |
|---|---|
| Berechnet: | C 57.61; H 8.35; N 6.11; S 13.98 |
| Gefunden: | C 57.72; H 8.46; N 5.98; S 13.99 |

### Beispiel 16:

Darstellung von 5-(2-(methylthic)ethyl)oxazolidin-2,4-dion (**15**) aus 2-Hydroxy-4-(methylthio)butansäureamid (**9**):

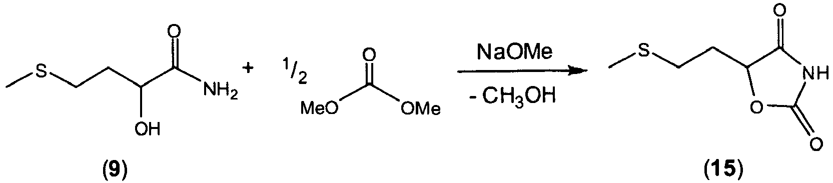

5.0 g (33.5 mol) 1-Hydroxy-3-(methylmercapto)-butansäureamid (**9**) wurden in einem 250 mL-Dreihalskolben in 50 mL Methanol suspendiert, 10 mL Dimethylcarbonat zugegeben und anschließend mit 9.05 g (168 mmol, 5.0 eq.) Natriummethylat versetzt. Das Reaktionsgemisch wurde zum Sieden erhitzt und 24 h lang bei dieser Temperatur unter Rückfluss gerührt. Die Reaktionslösung wurde abgekühlt und zweimal mit 100 mL kaltem Wasser versetzt und dreimal mit je 50 mL tert-Butylmethylether extrahiert. Die vereinigten org. Phase wurde über MgSO₄ getrocknet und am Rotationsverdampfer bei 15 mbar/40°C eingeengt und über Nacht in Kühlschrank aufbewahrt. Der kristallisierte Feststoff wurde mehrmals aus einer Mischung aus n-Hexan/EtOAc umkristallisiert. Nach der Filtration und Trocknung wurden letzte Lösungsmittelreste im Hochvakuum entfernt. Man erhielt 2.8 g (12.2 mmol, Ausbeute = 36.4%) 5-(2-(methylthio)ethyl)oxazolidin-2,4-dion (**15**) als farblosen Feststoff.

¹H-NMR von 5-(2-(methylthio)ethyl)oxazolidin-2,4-dion (**15**) (500 MHz, CDCl₃) : δ = 2.10 (s, 3H, SCH₃) ; 2.20-2.40 (m, 2H, CH-.) ; 2.60-2.80 (m, 2H, SCH₂) ; 5.0-5.2 (m, 1H, CH); 9.1 (bs, 1H, NH)

## Patentansprüche

1. Chemische Verbindung der allgemeinen Formel **I** oder **II**, 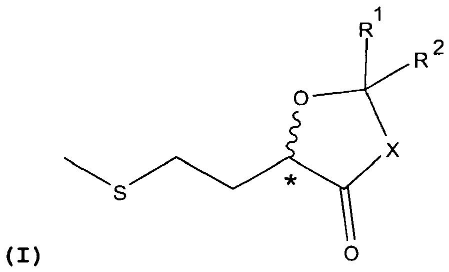 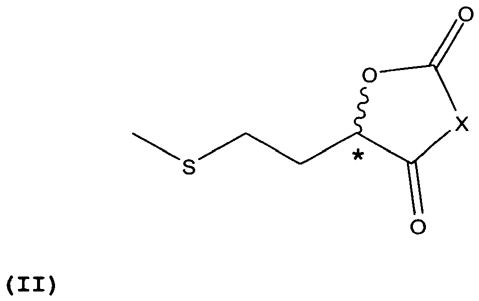 wobei X = O oder NR ist und R = H, ein ggf. verzweigtes C₁-C₆-Alkyl, C₃-C₆-Cycloalkyl, Aryl, insbesondere Phenyl, oder Aralkyl, insbesondere Benzyl, ist und wobei R¹, R² gleich oder verschieden ist und jeweils H, ein ggf. verzweigtes C₁-C₆-Alkyl, C₃-C₆-Cycloalkyl, Allyl-, Aryl, insbesondere Phenyl, oder Aralkyl, insbesondere Benzyl, oder R¹ und R² zusammen eine ggf. C₁-C₆-alkylsubstituierte C₂- bis C₆-Alkylengruppe ist.

2. Verbindung der Formel **I** gemäß Anspruch 1, **dadurch gekennzeichnet, dass** X = O ist.

3. Verbindung gemäß Anspruch 2, **dadurch gekennzeichnet, dass** R¹ = R² = H ist.

4. Verbindung gemäß Anspruch 2, **dadurch gekennzeichnet, dass** R¹ = H und R² = tert-Butyl ist.

5. Verbindung gemäß Anspruch 2, **dadurch gekennzeichnet, dass** R¹ = H und R² = Phenyl ist. ()

6. Verbindung gemäß Anspruch 2, **dadurch gekennzeichnet, dass** R¹ und R² jeweils ein ggf. verzweigtes C₁-C₆-Alkyl ist.

7. Verbindung gemäß Anspruch 6, **dadurch gekennzeichnet, dass** R¹ - R² = CH₃ ist.

8. Verbindung gemäß Anspruch 2, **dadurch gekennzeichnet, dass** R¹ und R² zusammen = (CH₂)₅ ist.

9. Verbindung der Formel **I** gemäß Anspruch 1, **dadurch gekennzeichnet, dass** X = NH ist.

10. Verbindung gemäß Anspruch 9, **dadurch gekennzeichnet, dass** R¹ = R² = H ist.

11. Verbindung gemäß Anspruch 9, **dadurch gekennzeichnet, dass** R¹ = H und R² = Phenyl ist.

12. Verbindung gemäß Anspruch 9, **dadurch gekennzeichnet, dass** R¹ und R² jeweils ein ggf. verzweigtes C₁-C₆-Alkyl ist.

13. Verbindung gemäß Anspruch 12, **dadurch gekennzeichnet, dass** R¹ = R² = CH₃ ist.

14. Verbindung gemäß Anspruch 12, **dadurch gekennzeichnet, dass** R¹ = CH₃ und R² = C₂H₅ ist.

15. Verbindung gemäß Anspruch 2, **dadurch gekennzeichnet, dass** R¹ und R² zusammen = (CH₂)₅ ist.

16. Verbindung der Formel **II** gemäß Anspruch 1, **dadurch gekennzeichnet, dass** X = O ist.

17. Verbindung der Formel **II** gemäß Anspruch 1, **dadurch gekennzeichnet, dass** X = NH ist.

18. Verwendung von Verbindungen gemäß Anspruch 1- 17 zur Ernährung von Nutztieren.

19. Verwendung gemäß Anspruch 18 zur Ernährung von Geflügel, Schweinen, Wiederkäuern, Fischen oder Krustentieren.

20. Futtermischungen zur Ernährung von Nutztieren enthaltend mindestens eine Verbindung gemäß Anspruch 1- 17.

21. Verwendung von Verbindungen gemäß Anspruch 1- 17 zur Herstellung von Futtermischungen zur Ernährung von Nutztieren.

22. Verfahren zur Herstellung von Verbindungen der Formel **I** gemäß Anspruch 1, **dadurch gekennzeichnet, dass** man eine Verbindung der allgemeinen Formel **III** 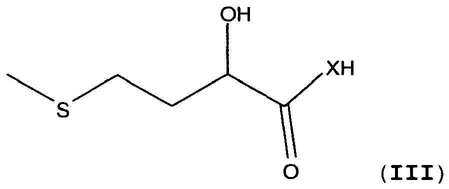 mit einer Carbonylverbindung R¹R²C=O in freier oder in acetalisierter Form ggf. in Anwesenheit eines Lösungsmittels umsetzt, wobei X, R¹ und R² jeweils die in Anspruch 1 angegebene Bedeutung haben.

23. Verfahren gemäß Anspruch 22, **dadurch gekennzeichnet, dass** man eine Lewissäure oder eine Brönstedt-Säure als Katalysator verwendet.

24. Verfahren gemäß Anspruch 23, **dadurch gekennzeichnet, dass** man HCl, H₂SO₄, *p*-Toluolsulfonsäure, CF₃SO₃H, ZnCl₂, CuSO₄, FeCl₃, AlCl₃, MgCl₂, MgBr₂ als Katalysator verwendet.

25. Verfahren gemäß Anspruch 22 - 24, **dadurch gekennzeichnet, dass** man das Dimethyl- oder Diethylacetal der Verbindung R¹R²C=O einsetzt.

26. Verfahren gemäß einem der Ansprüche 22 - 25, **dadurch gekennzeichnet, dass** während der Reaktion entstehendes Wasser oder entstehender Alkohol entfernt wird.

27. Verfahren zur Herstellung von Verbindungen der Formel **II** gemäß Anspruch 1, **dadurch gekennzeichnet, dass** man eine Verbindung der allgemeinen Formel **III** 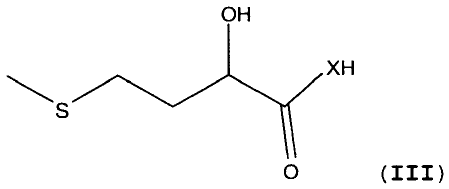 mit einem Kohlensäurederivat X¹X²C=O umsetzt, wobei X¹ und X² gleich oder verschieden sind und unabhängig voneinander Chlor oder OCCl₃, OCH₃, OCH₂CH₃, oder über den Stickstoff gebundenes Imidazolyl oder Triazolyl sein können.

28. Verfahren gemäß Anspruch 27, **dadurch gekennzeichnet, dass** X¹ = Cl und X² = OCCl₃ ist.

29. Verfahren gemäß einem der Ansprüche 27 oder 28, **dadurch gekennzeichnet, dass** die Reaktion sauer oder basisch katalysiert durchgeführt wird.

30. Verfahren zur Herstellung von Verbindungen der Formel I gemäß Anspruch 1 mit X = NH, **dadurch gekennzeichnet, dass** man das Hydroxynitril der Formel **IV** 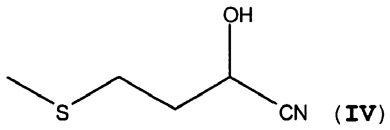 mit einer Carbonylverbindung R¹R²C=O in Gegenwart von Säure und einem Carbonsäureanhydrid umsetzt, wobei R¹ und R² die in Anspruch 1 angegebene Bedeutung haben.

31. Verfahren gemäß Anspruch 30, **dadurch gekennzeichnet, dass** als Säure Schwefelsäure und/oder Essigsäure und als Carbonsäureanhydrid Essigsäureanhydrid verwendet werden.

## Claims

1. Chemical compound of the general formula **I** or **II**, 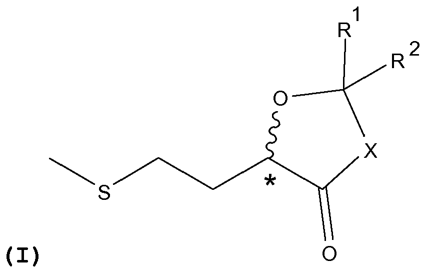 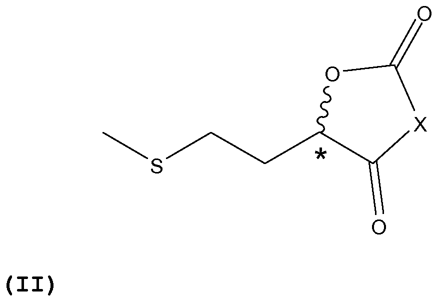 wherein X = 0 or NR, and R = H, an optionally branched C₁-C₆-alkyl, C₃-C₆-cycloalkyl, aryl, in particular phenyl, or aralkyl, in particular benzyl, and wherein R¹, R², are identical or different and in each case H, an optionally branched C₁-C₆-alkyl, C₃-C₆-cycloalkyl, allyl, aryl, in particular phenyl, or aralkyl, in particular benzyl, or R¹ and R² together are an optionally C₁-C₆-alkyl substituted C₂- to C₆-alkylene group.

2. Compound of the formula **I** according to Claim 1, **characterized in that** X = O.

3. Compound according to Claim 2, **characterized in that** R¹ = R² = H.

4. Compound according to Claim 2, **characterized in that** R¹ = H and R² = *tert*-butyl.

5. Compound according to Claim 2, **characterized in that** R¹ = H and R² = phenyl.

6. Compound according to Claim 2, **characterized in that** R¹ and R² are each an optionally branched C₁-C₆-alkyl.

7. Compound according to Claim 6, **characterized in that** R¹ - R² - CH₃.

8. Compound according to Claim 2, **characterized in that** R¹ and R² together = (CH₂)₅.

9. Compound of the formula **I** according to Claim 1, **characterized in that** X = NH.

10. Compound according to Claim 9, **characterized in that** R¹ = R² = H.

11. Compound according to Claim 9, **characterized in that** R¹ = H and R² = phenyl.

12. Compound according to Claim 9, **characterized in that** R¹ and R² are each an optionally branched C₁-C₆-alkyl.

13. Compound according to Claim 12, **characterized in that** R¹ - R² - CH₃.

14. Compound according to Claim 12, **characterized in that** R¹ = CH₃ and R² = C₂H₅.

15. Compound according to Claim 2, **characterized in that** R¹ and R² together = (CH₂)₅.

16. Compound of the formula **II** according to Claim 1, **characterized in that** X = O.

17. Compound of the formula **II** according to Claim 1, **characterized in that** X = NH.

18. Use of compounds according to Claim 1-17 for the nutrition of farm animals.

19. Use according to Claim 18 for the nutrition of poultry, pigs, ruminants, fish or crustacea.

20. Feed mixture for the nutrition of farm animals which comprises at least one compound according to Claim 1- 17.

21. Use of compounds according to Claim 1- 17 for production of feed mixtures for the nutrition of farm animals.

22. Process for producing compounds of the formula **I** according to Claim 1, **characterized in that** a compound of the general formula **III** 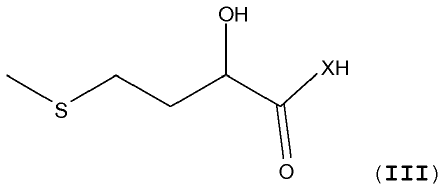 is reacted with a carbonyl compound R¹R²C=O in free or acetalated form if appropriate in the presence of a solvent, wherein X, R¹ and R² each have the meaning given in Claim 1.

23. Process according to Claim 22, **characterized in that** a Lewis acid or a Brönstedt acid is used as catalyst.

24. Process according to Claim 23, **characterized in that** use is made of HCl, H₂SO₄, *p*-toluenesulfonic acid, CF₃SO₃H, ZnCl₂, CuSO₄, FeCl₃, AlCl₃, MgCl₂, MgBr₂ as catalyst.

25. Process according to Claim 22-24, **characterized in that** use is made of the dimethylacetal or diethylacetal of the compound R¹R²C=O.

26. Process according to one of Claims 22-25, **characterized in that** water or alcohol which is formed during the reaction is removed.

27. Process for producing compounds of the formula **II** according to Claim 1, **characterized in that** a compound of the general formula **III** 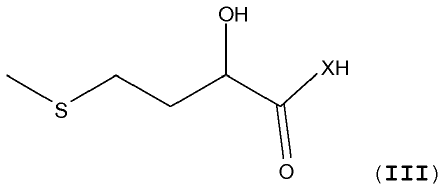 is reacted with a carbonic acid derivative X¹X²C=O, wherein X¹ and X² are identical or different and independently of one another can be chlorine or OCCl₃, OCH₃, OCH₂CH₃, or imidazolyl or triazolyl bound via the nitrogen.

28. Process according to Claim 27, **characterized in that** X¹ = Cl and X² = OCCl₃.

29. Process according to one of Claims 27 or 28, **characterized in that** the reaction is carried out under acid or base catalysis.

30. Process for producing compounds of the formula I according to Claim 1 where X = NH, **characterized in that** the hydroxynitrile of the formula **IV** 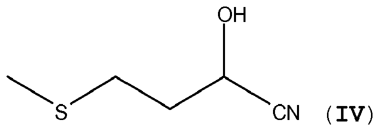 is reacted with a carbonyl compound R¹R²C=O in the presence of acid and a carboxylic anhydride, wherein R¹ and R² have the meaning given in Claim 1.

31. Process according to Claim 30, **characterized in that** the acid is sulfuric acid and/or acetic acid and the carboxylic anhydride is acetic anhydride.

## Revendications

1. Composé chimique de formule générale **I** ou **II**, 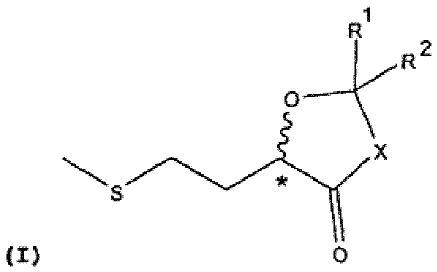 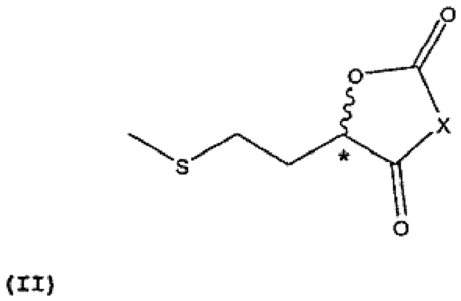 où X = O ou NR et R = H, un groupe alkyle en C₁-C₆ éventuellement ramifié, cycloalkyle en C₃-C₆, aryle, en particulier phényle, ou aralkyle, en particulier benzyle, et où R¹, R² sont identiques ou différents et représentent chacun H, un groupe alkyle en C₁-C₆ éventuellement ramifié, cycloalkyle en C₃-C₆, allyle, aryle, en particulier phényle, ou aralkyle, en particulier benzyle, ou R¹ et R² forment ensemble un groupe alkylène en C₂-C₆ éventuellement substitué par alkyle en C₁-C₆.

2. Composé de formule **I** selon la revendication 1, **caractérisé en ce que** X = O.

3. Composé selon la revendication 2, **caractérisé en ce que** R ¹ = R ² = H_{.}

4. Composé selon la revendication 2, **caractérisé en ce que** R¹ = H et R² = tert-butyle.

5. Composé selon la revendication 2, **caractérisé en ce que** R¹ = H et R² = phényle.

6. Composé selon la revendication 2, **caractérisé en ce que** R¹ et R² représentent chacun un groupe alkyle en C₁-C₆ éventuellement ramifié.

7. Composé selon la revendication 6, **caractérisé en ce que** R¹ = R² = CH₃.

8. Composé selon la revendication 2, **caractérisé en ce que** R¹ et R² forment ensemble un groupement (CH₂)₅.

9. Composé de formule **I** selon la revendication 1, **caractérisé en ce que** X = NH.

10. Composé selon la revendication 9, **caractérisé en ce que** R ¹ = R² = H.

11. Composé selon la revendication 9, **caractérisé en ce que** R¹ - H et R² = phényle.

12. Composé selon la revendication 9, **caractérisé en ce que** R¹ et R² représentent chacun un groupe alkyle en C₁-C₆ éventuellement ramifié.

13. Composé selon la revendication 12, **caractérisé en ce que** R¹ = R² = CH₃.

14. Composé selon la revendication 12, **caractérisé en ce que** R¹ = CH₃ et R² = C₂H₅.

15. Composé selon la revendication 2, **caractérisé en ce que** R¹ et R² forment ensemble un groupement (CH₂)₅.

16. Composé de formule **II** selon la revendication 1, **caractérisé en ce que** X = O.

17. Composé de formule **II** selon la revendication 1, **caractérisé en ce que** X = NH.

18. Utilisation de composés selon l'une quelconque des revendications 1 à 17, pour l'alimentation d'animaux de rapport.

19. Utilisation selon la revendication 18, pour l'alimentation de volailles, porcs, ruminants, poissons ou crustacés.

20. Compositions d'aliments pour animaux destinées à l'alimentation d'animaux de rapport, contenant au moins un composé selon l'une quelconque des revendications 1 à 17.

21. Utilisation de composés selon l'une quelconque des revendications 1 à 17, pour la production de compositions d'aliments pour animaux destinées à l'alimentation d'animaux de rapport.

22. Procédé pour la préparation de composés de formule **I** selon la revendication 1, **caractérisé en ce qu'**on fait réagir un composé de formule générale **III** 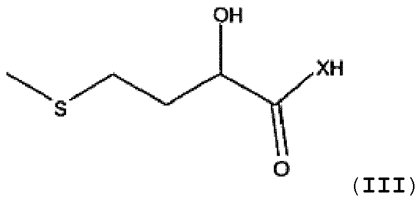 avec un composé carbonyle R¹R²C=O sous forme libre ou sous forme acétalisée, éventuellement en présence d'un solvant, X, R¹ et R² ayant chacun la signification indiquée dans la revendication 1.

23. Procédé selon la revendication 22, **caractérisé en ce qu'**on utilise comme catalyseur un acide de Lewis ou un acide de Brönsted.

24. Procédé selon la revendication 23, **caractérisé en ce qu'**on utilise comme catalyseur HCl, H₂SO₄, l'acide p-toluènesulfonique, CF₃SO₃H, ZnCl₂, CuSO₄, FeCl₃, AlCl₃, MgCl₂, MgBr₂.

25. Procédé selon l'une quelconque des revendications 22 à 24, **caractérisé en ce qu'**on utilise le diméthyl- ou diéthylacétal du composé R¹R²C=O.

26. Procédé selon l'une quelconque des revendications 22 à 25, **caractérisé en ce que** pendant la réaction on élimine l'eau formée ou l'alcool formé.

27. Procédé pour la préparation de composés de formule **II** selon la revendication 1, **caractérisé en ce qu'**on fait réagir un composé de formule générale **III** 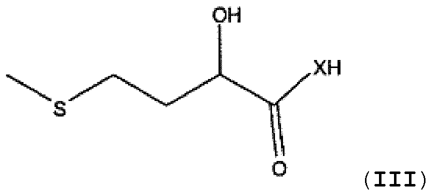 avec un dérivé d'acide carbonique X¹X²C=O, X¹ et X² étant identiques ou différents et pouvant représenter, indépendamment l'un de l'autre, un atome de chlore ou OCCl₃ OCH₃, OCH₂CH₃, ou un groupe imidazolyle ou triazolyle lié par l'atome d'azote.

28. Procédé selon la revendication 27, **caractérisé en ce que** X¹ = Cl et X² = OCCl₃.

29. Procédé selon la revendication 27 ou 28, **caractérisé en ce qu'**on effectue la réaction avec catalyse acide ou basique.

30. Procédé pour la préparation de composés de formule I selon la revendication 1, où X = NH, **caractérisé en ce qu'**on fait réagir l'hydroxynitrile de formule **IV** 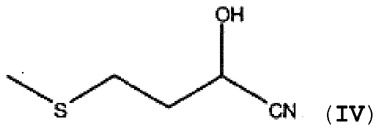 avec un composé carbonyle R¹R²C=O en présence d'acide et d'un anhydride d'acide carboxylique, R¹ et R² ayant la signification indiquée dans la revendication 1.

31. Procédé selon la revendication 30, **caractérisé en ce qu'**on utilise comme acide l'acide sulfurique et/ou l'acide acétique et en tant qu'anhydride d'acide carboxylique l'anhydride acétique.
